## Europäisches Patentamt

## European Patent Office

## Office européen des brevets

(19)

(11) Veröffentlichungsnummer: **0 041 683**
**B1**

(12) # EUROPÄISCHE PATENTSCHRIFT

(45) Veröffentlichungstag der Patentschrift:
10.10.84

(51) Int. Cl.³: **G 01 N 33/72**

(21) Anmeldenummer: 81104200.1

(22) Anmeldetag: 02.06.81

(54) Prüfmittel zum Nachweis kuppelnder Verbindungen sowie ein Verfahren zu seiner Herstellung.

(30) Priorität: 06.06.80 DE 3021305

(43) Veröffentlichungstag der Anmeldung:
16.12.81 Patentblatt 81/50

(45) Bekanntmachung des Hinweises auf die Patenterteilung:
10.10.84 Patentblatt 84/41

(84) Benannte Vertragsstaaten:
AT BE CH DE FR IT LI

(56) Entgegenhaltungen:
EP - A - 0 008 233
EP - A - 0 009 164
EP - A - 0 022 240
EP - A - 0 025 889
DE - A - 2 623 087
DE - A - 2 728 236
DE - B - 2 240 357
US - A - 4 246 133

H. BEYER, W. WALTER, LEHRBUCH DER
ORGANISCHEN CHEMIE, STUTTGART, 1981, Seiten
167,168

(73) Patentinhaber: BEHRINGWERKE
AKTIENGESELLSCHAFT, Postfach 1140,
D-3550 Marburg/Lahn (DE)

(72) Erfinder: Kohl, Helmut, Dr., Goethestrasse 32,
D-3552 Wetter (DE)

(74) Vertreter: Meyer-Dulheuer, Karl-Hermann, Dr. et al,
HOECHST Aktiengesellschaft Zentrale Patentabteilung
Postfach 80 03 20, D-6230 Frankfurt/Main 80 (DE)

**Beschreibung**

Die Erfindung betrifft ein Prüfmittel zum Nachweis kuppelnder Verbindungen in Körperflüssigkeiten, das im wesentlichen aus einer adsorbierenden Matrix besteht, welche ein Diazoniumsalz als Reagens und ein Phosphoniumsalz enthält. Die Erfindung betrifft auch die Herstellung dieses Prüfmittels.

Die analytische Bestimmung kuppelnder Verbindungen, wie z. B. Bilirubin oder Urobilinogen, in Körperflüssigkeiten mit Diazoniumsalzen ist eine gebräuchliche Methode. Beispiele hierfür werden z. B. in der DE-OS 2 623 087 oder in der DE-PS 2 936 745 gegeben.

Bei Leberschädigungen und Gallenverschlüssen tritt schon frühzeitig Bilirubin im Urin auf. Es kann dort mittels Kupplungsreaktion mit einem Diazoniumsalz bestimmt werden. Bei diesen Nachweisreaktionen entstehen empfindliche, relativ schlecht erkennbare Farben.

Heute ist es üblich, schnelle, orientierende Bestimmungen von Bilirubin im Serum bzw. Urin mit Schnelltesten durchzuführen. Diese sind Prüfmittel, bestehend aus einer flächenbildenden Trägermatrix, die mit Diazoniumsalzen und weiteren Hilfsstoffen imprägniert wurde. Im allgemeinen werden heute unterschiedliche Schnellteste zu Kombinationstesten zusammengefaßt. Ein Kombinationsteststreifen kann z. B. Testbezirke zur Erkennung von Glucose, Bilirubin, Protein und Keton-Körpern im Urin enthalten. Kombinationsteststreifen solcher Art werden u. a. unter dem Warenzeichen RAPIGNOST® vertrieben.

Im allgemeinen sind die Schnellteststreifen äußerst labil; so zersetzen die Reagenzien sich schon durch Luftfeuchtigkeit. Bei Kombinationsteststreifen ist es auch möglich, daß sich benachbarte Testbezirke gegenseitig negativ beeinflussen. So wurde festgestellt, daß sich ein Bilirubin-Testpapier, enthaltend u. a. Dichlorbenzoldiazoniumsalz, sehr leicht verfärbt, wenn es bei Raumtemperatur einer Luftfeuchtigkeit von >60% ausgesetzt wird bzw. wenn es gemeinsam mit stark basisch gepufferten Testbezirken verarbeitet wird. Es entstehen dann jeweils gelb oder rot gefärbte Bilirubin-Testbezirke. Diese Verfärbungen können aber auch während der Lagerung der Schnelldiagnostika auftreten. Für ihren diagnostischen Gebrauchswert ist es aber entscheidend, daß sich die farblosen Papiere nicht verfärben, da nur so die bei positiver Bilirubin-Reaktion auftretenden schwachen Farbreaktionen wahrgenommen werden können.

Die üblicherweise verwendeten Diazoniumsalze sind äußerst labile Verbindungen. Sie werden in stabilisierter Form gehandelt. Stabilisatoren können u. a. sein: Sulfonsäuren oder Lewis-Säuren, wie z. B. AlCl$_3$, SnCl$_2$ oder BF$_4$.

In der DE-OS 2 007 013 werden Sulfonsäuren als Stabilisatoren für Diazoniumsalze auf Teststreifen beansprucht. Die Zusätze dienen dazu, einen vorgegebenen Gehalt von Diazoniumsalz auf dem Teststreifen aufrechtzuerhalten. In der DE-OS 2 012 558 wird die Verwendung von Lewis-Säuren beschrieben. Diese sollen einen farbverstärkenden Effekt bewirken.

Die Verwendung von Phosphorsäurediestern wird in der DE-AS 2 240 357 beansprucht. Diese Verbindungen haben, wie auch eigene Untersuchungen bestätigen, einen aktivierenden Einfluß auf die Bilirubin-Bestimmung. Stabilisierende Wirkungen werden nicht erwähnt.

Das verwendete Diazoniumsalz wird mit NaHSO$_4$ stabilisiert und enthält zusätzlich noch ein BF$_4$$^-$-Anion. Ein Testpapier kann so ohne Qualitätsverluste mehrere Jahre lang gelagert werden. Wird das Bilirubin-Testpapier allerdings produktionsbedingt gemeinsam mit anderen Testpapieren, besonders mit Keton-Testpapier, verarbeitet, verfärbt es sich trotz großer Vorsichtsmaßnahmen sehr schnell rosa.

Ziel der vorliegenden Erfindung war daher ein Stabilisator für Schnelldiagnostika zum Nachweis kuppelnder Verbindungen in Körperflüssigkeiten. Der Stabilisator sollte die Schnelldiagnostika vor Verfärbungen schützen, die bei ihrer Herstellung bzw. Lagerung auftreten könnten.

Die gestellte Aufgabe wird dadurch gelöst, daß als Stabilisator für Schnelldiagnostika zum Nachweis kuppelnder Verbindungen in Körperflüssigkeiten ein Phosphoniumsalz verwendet wird.

Gegenstand der Erfindung ist ein Prüfmittel zum Nachweis kuppelnder Verbindungen in Körperflüssigkeiten, bestehend im wesentlichen aus einer adsorbierenden Matrix, die mit einem Diazoniumsalz und ggfs. einem Stabilisator hierfür, imprägniert ist, dadurch gekennzeichnet, daß es ein Phosphoniumsalz enthält. Die Erfindung umfaßt auch die Herstellung dieses Prüfmittels.

Als Stabilisator wird alkyliertes und/oder aryliertes Phosphoniumsalz, das evtl. ggfs. gleichzeitig mit Alkyl- und Arylgruppen substituiert sein kann, verwendet. Bevorzugt werden alkylierte Phosphoniumverbindungen, die als Halogenide, vor allem als Bromide vorliegen, insbesondere solche Tetraalkylphosphoniumsalze. Besonders bevorzugt wird das Ethyltrioctylphosphoniumbromid.

Weitere geeignete Stabilisatoren sind z. B. Tetrabutylphosphoniumbromid, Hexadecyltributylphosphoniumbromid oder Phenyltributylphosphoniumbromid.

Die Auswahl des Anions ist jedoch nicht kritisch, es können beliebige Anionen wie z. B. Halogenid, Hydroxid, Sulfat oder Sulfonat verwendet werden.

Hergestellt können die erfindungsgemäßen Stabilisatoren gemäß »Methoden der Organischen Chemie, Houben-Weyl, Band XII, 1, S. 79 ff., werden.

Das Prüfmittel gemäß der Erfindung besteht aus einer adsorbierenden Trägermatrix, in welchem das Reagenz zum Nachweis von mit Diazoniumsalz kuppelnden Verbindungen und ein Phosphoniumsalz eingelagert sind. Als Träger-

matrix können alle saugfähigen flächenförmigen Gebilde natürlichen oder synthetischen Ursprungs verwendet werden, wie z. B. Vliese, Papiere, Asbest oder Folien aus Polymeren.

Die Zubereitung des Prüfmittels kann unterschiedlich erfolgen. So können die Phosphoniumsalze vor oder nach dem jeweiligen Diazoniumsalz auf das Testpapier aufgetragen werden. Es ist auch möglich, sie gemeinsam mit dem Diazoniumsalz auf das Schnelldiagnostikum aufzutragen. Bewährt hat sich ein Verfahren, bei dem zunächst ein Indikatorrohpapier mit einem Diazoniumsalz und weiteren Hilfsmitteln wie z. B. Puffer-Salzen, Stabilisatoren und Netzmitteln in Wasser oder mit Wasser mischbaren organischen Lösungsmitteln imprägniert wird und dann in einem zweiten Schritt das Phosphoniumsalz in einem organischen Lösungsmittel wie Alkohol (z. B. n-Propanol) oder polaren aprotischen Lösungsmitteln (z. B. Äthylenglykol) aufgetragen wird. Bei der Wahl des Lösungsmittels für die Zweitimprägnierung ist darauf zu achten, daß dieses die während der Erstimprägnierung applizierten Komponenten nicht mehr von der Trägermatrix löst.

Das bevorzugte Verfahren zur Zubereitung des vorliegenden Prüfmittels umfaßt somit mehrere Stufen, wobei man mit der beschriebenen Imprägnierung der adsorbierenden Matrix beginnt. Nach dem Trocknen kann der Träger auf Papier auf einen Kunststoffilm laminiert werden.

Die Menge des jeweiligen Stabilisators ist nicht kritisch, sie wird nur durch seine Löslichkeit in dem ausgewählten Lösungsmittel begrenzt. So kann z. B. die jeweilige Konzentration des Stabilisators zwischen 0,1% und 10% liegen, bevorzugt werden aber Konzentrationen zwischen 0,4% und 5%.

Bei Verwendung der Phosphoniumverbindungen bleibt eine nicht tolerierbare Verfärbung der Bilirubin-Papiere aus. Stabilisierende Einflüsse der Phosphoniumsalze auf den Gehalt an Diazoniumsalz wurden nicht beobachtet. Die Testpapiere sind auch so ausreichend stabil. Den Nachweis von Bilirubin aktivieren sie nicht.

Bei der Verwendung des vorliegenden Prüfmittels wird dieses kurz in die zu untersuchende Flüssigkeit eingetaucht und sofort herausgenommen. Abhängig von dem Gehalt der Flüssigkeit an kuppelnden Verbindungen entwickelt sich eine spezifische Färbung.

Bei vergleichenden Untersuchungen mit Schnelldiagnostika gemäß dem Stand der Technik zeigt sich überraschend der unerwartet große Vorteil der Erfindung.

Für einen Vergleichsversuch wurden zwei Testpapiere hergestellt. Das Testpapier wurde entsprechend dem Beispiel 1 mit einem Diazoniumsalz in Lösung 1 imprägniert, zur Herstellung von Testpapier 2 wurde das Testpapier 1 nachträglich noch mit einer Lösung von 10 g/l Ethyl-trioctylphosphoniumbromid in n-Propanol (Lösung 2) behandelt. Nach dem Trocknen waren beide Papiere farblos.

Beim ersten Versuch wurden die Testpapiere 1 und 2 zwölf Stunden bei 25°C in einem Klimaraum bei 70% rel. Luftfeuchtigkeit gelagert. Das Testpapier 1 war anschließend gelb gefärbt, das Testpapier 2 hatte seine Farbe nicht geändert. In einem weiteren Versuch wurden die Testpapiere 1 und 2 zusammen mit stark basisch gepufferten Testpapieren zur Bestimmung von Ketonkörpern auf einem Kunststoffträger aufgesiegelt. Dieser wurde zu Teststreifen zerschnitten, die bei 70% und 10% rel. Luftfeuchtigkeit und bei 50°C gelagert wurden. Bei allen Lagerbedingungen verfärbten sich die Testpapiere 1 rosa, die Testpapiere 2 blieben farblos. Die Testpapiere 1 waren für die Bestimmung von Bilirubin nicht mehr geeignet, mit den Testpapieren 2 konnten noch Spuren von Bilirubin im Bereich um 0,4 mg% deutlich nachgewiesen werden.

Mit dem folgenden Beispiel wird die Erfindung näher erläutert:

### Beispiel

#### Lösung 1

In 300 ml Wasser werden gelöst

0,7 g Pyrazol-3-Diazoniumtetrafluoroborat
40 g m-Phosphorsäure
20 g Natriumhydrogensulfat
5 g Dodecylbenzolsulfonat

#### Lösung 2

In 100 ml n-Propanol wird gelöst

1 g Ethyl-trioctylphosphoniumbromid

Papier Schleicher und Schüll 2316 wird mit Lösung 1 imprägniert und getrocknet (Testpapier 1).

Das trockene Testpapier 1 wird mit der Lösung 2 behandelt (Testpapier 2). Beide Papiere zeigen die beschriebenen Effekte.

Es kann aber auch ein Indikatorrohpapier zunächst mit der Lösung 2 behandelt werden und dann mit der Lösung 1. Dieses Testpapier unterscheidet sich nicht von dem oben erwähnten Testpapier 2.

### Patentansprüche

1. Prüfmittel zum Nachweis kuppelnder Verbindungen in Körperflüssigkeiten, bestehend im wesentlichen aus einer adsorbierenden Matrix, die mit einem Diazoniumsalz, ggfs. einem Stabilisator hierfür, imprägniert ist, dadurch gekennzeichnet, daß es ein Phosphoniumsalz enthält.

2. Prüfmittel nach Anspruch 1, dadurch gekennzeichnet, daß das Phosphoniumsalz mit Alkyl-, Aryl- oder Alkyl- und Arylgruppen substituiert ist.

3. Prüfmittel nach Anspruch 1, dadurch gekennzeichnet, daß das Phosphoniumsalz Ethyl-trioctylphosphoniumbromid ist.

4. Verfahren zur Herstellung eines Prüfmittels nach einem der Ansprüche 1 bis 3, dadurch gekennzeichnet, daß man die adsorbierende Matrix zunächst mit einem Diazoniumsalz und ggfs. weiteren Hilfsmitteln und danach mit einem Phosphoniumsalz imprägniert.

## Claims

1. A test agent for the detection of coupling compounds in body fluids, consisting essentially of an adsorbent matrix, which is impregnated with a diazonium salt and, if appropriate, a stabilizer for this salt, which test agent contains a phosphonium salt.

2. A test agent as claimed in claim 1, in which the phosphonium salt is substituted by alkyl or aryl or alkyl and aryl groups.

3. A test agent as claimed in claim 1, wherein the phosphonium salt is ethyl-trioctylphosphonium bromide.

4. A process for the preparation of a test agent as claimed in any of claims 1 to 3, which comprises impregnating the adsorbent matrix first with a diazonium salt and, if appropriate, other auxiliaries and then with a phosphonium salt.

## Revendications

1. Agent d'essai pour la détection de composés copulants dans des liquides corporels, consistant essentiellement en une matrice adsorbante qui est imprégnée avec un sel de diazonium et, éventuellement, un stabilisant approprié, caractérisé en ce qu'il contient un sel de phosphonium.

2. Agent d'essai selon la revendication 1, caractérisé en ce que le sel de phosphonium est substitué avec des groupes alkyle, aryle ou alkyle et aryle.

3. Agent d'essai selon la revendication 1, caractérisé en ce que le sel de phosphonium est le bromure d'éthyl-trioctyl-phosphonium.

4. Procédé pour la préparation d'un agent d'essai selon l'une des revendications 1 à 3, caractérisé en ce qu'on imprègne la matrice adsorbante d'abord avec un sel de diazonium et éventuellement d'autres adjuvants, puis avec un sel de phosphonium.